(19) Europäisches Patentamt / European Patent Office / Office européen des brevets

(11) **EP 4 321 244 A1**

(12) **EUROPEAN PATENT APPLICATION**
published in accordance with Art. 153(4) EPC

(43) Date of publication:
**14.02.2024 Bulletin 2024/07**

(21) Application number: **22784134.3**

(22) Date of filing: **08.04.2022**

(51) International Patent Classification (IPC):
**B01J 8/24** (2006.01)          **C07C 253/26** (2006.01)
**C07C 255/08** (2006.01)          **F28D 13/00** (2006.01)

(52) Cooperative Patent Classification (CPC):
**B01J 8/24; C07C 253/26; C07C 255/08; F28D 13/00**

(86) International application number:
**PCT/CN2022/085769**

(87) International publication number:
**WO 2022/214068 (13.10.2022 Gazette 2022/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.04.2021 CN 202110384148
29.01.2022 CN 202210111952**

(71) Applicants:
• **China Petroleum & Chemical Corporation
Beijing 100728 (CN)**
• **Shanghai Research Institute of Petrochemical Technology SINOPEC
Pudong New Area
Shanghai 201208 (CN)**

(72) Inventors:
• **ZHAO, Le
Shanghai 201208 (CN)**
• **WU, Lianghua
Shanghai 201208 (CN)**

(74) Representative: **karo IP
karo IP Patentanwälte
Kahlhöfer Rößler Kreuels PartG mbB
Postfach 32 01 02
40416 Düsseldorf (DE)**

(54) **HEAT REMOVAL PIPE GROUP, AND APPLICATION THEREOF IN TEMPERATURE CONTROL OF FLUIDIZED-BED REACTOR AND FABRICATION OF UNSATURATED NITRILES**

(57) The present application relates to a heat removal tube set, and application of the same for controlling the temperature of a fluidized bed reactor and for producing an unsaturated nitrile. The heat removal tube set comprises at least one first heat removal tube and at least one second heat removal tube, wherein the ratio of the total circumference Lb of the outer contours of all of the straight pipes b of the second heat removal tube on the cross section to the total circumference La of the outer contours of all of the straight pipes a of the first heat removal tube on the cross section is greater than 1 and less than 1.25. According to the present application, where the first heat removal tube and the second heat removal tube are switched coordinatively in a paired manner, the reaction temperature can be maintained substantially constant.

Fig. 3

EP 4 321 244 A1

**Description**

**Technical Field**

The present application relates to a heat removal tube set particularly suitable for use in a fluidized bed reactor. The present application further relates to the application of the heat removal tube set in the temperature control of a fluidized bed reactor and the production of unsaturated nitrile.

**Background Art**

Acrylonitrile is an important chemical raw material for petrochemical industry. The one-step method for producing acrylonitrile by propylene ammoxidation is commonly adopted in various countries in the world, namely, under the action of a fluidized bed ammoxidation catalyst and under a certain reaction temperature and pressure, the propylene is subjected to ammoxidation to generate acrylonitrile, and at the same time generate byproducts such as acetonitrile, hydrocyanic acid and the like, and deep oxidation products like CO, $CO_2$ are also generated. The reaction is strongly exothermic and is accompanied by the generation of a large amount of heat.

Typical internals of acrylonitrile fluidized bed reactor include a propylene-ammonia distributor, an air distribution plate, a heat removal tube (also known as cooling coil) and a cyclone separator, wherein the heat removal tube and the dipleg of the cyclone separator are disposed in the catalyst bed as vertical members of the fluidized bed. The heat removal tube can remove a large amount of generated reaction heat out of the reaction system in time and maintain the reaction temperature in a stable state, and the cyclone separator captures the catalyst carried by the gas moving upwards and returns the catalyst to the catalyst bed through the dipleg so as to reduce the loss of the catalyst.

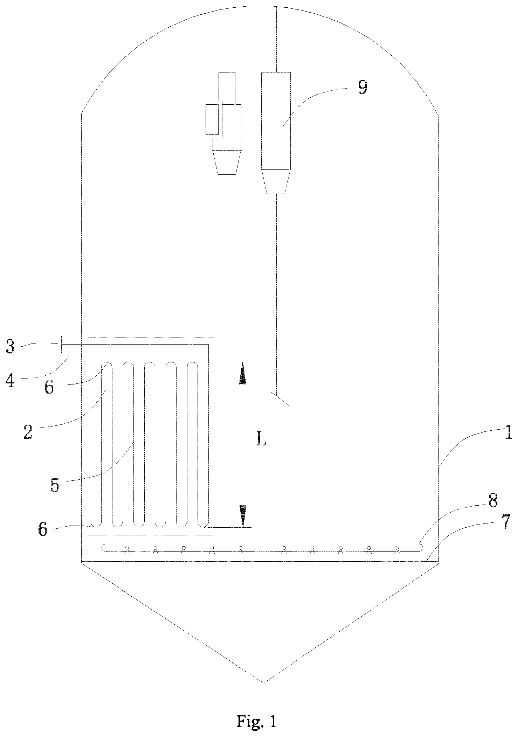

Fig. 1 shows an acrylonitrile fluidized bed reactor, of which the internals mainly include: a distribution plate for oxygen-containing gas, a propylene-ammonia distributor, a heat removal tube and a cyclone separator. In an existing acrylonitrile reactor shown in Fig. 1, 85% or more of the total heat removal tubes are in operation, i.e., those heat removal tubes are filled with a cold heat removal medium relative to the reaction temperature, and the reaction temperature is maintained stable by heat exchange with the heat removal medium. In addition, the purpose of finely adjusting the temperature of the reactor is typically achieved by switching heat removal tubes during the reaction process.

**Disclosure of the Invention**

The inventors of the present application have found that, in a fluidized bed reactor, the heat transfer efficiency near the reactor wall (periphery) is slightly lower than at the center. As a result, depending on their position (center or periphery) in the fluidized bed reactor, the heat quantity removed by different heat removal tubes is slightly different even with the same heat transfer area, and the influence thereof on the reaction temperature is also slightly different. Different heat removal tubes (including different heat removal tubes at different positions) need to be switched regularly during operation to achieve the purposes of descaling and the like, so that the problem of reaction temperature fluctuation (with an amplitude of 1-3 °C) is caused. In order to maintain the stability of the reaction temperature, the reaction heat released may be controlled to be equivalent to the heat removal capacity of the heat removal tube in work by increasing or decreasing the feed rate of the feed gas, namely increasing or decreasing the reaction heat, but a change of the feed rate of the feed gas of about $\pm 1\%$ or higher may be caused, so that unstable factors may be increased due to the change of the operation load of the equipment; or the steam drum pressure can be adjusted, but the fluctuation of the drum pressure may increase the operation difficulty of equipment requiring the steam turbine, such as air compressor, ice maker and the like.

The inventors of the present application have also found that in the acrylonitrile fluidized bed reactor involved in the present application, when the equipment is in full-load operation, the parameters, such as the feed rate of the feed gas, the reaction temperature, the reaction pressure, the drum pressure and the like, are known, and the heat removal capacity of the heat removal tubes in the fluidized bed reactor can be estimated, and the heat removal areas of different heat removal tubes at different positions can be matched to compensate the difference of the heat quantity removed. The present application has been completed based on this finding.

Specifically, the present application relates to the technical solutions of the following aspects:

1. A heat removal tube set (particularly a heat removal water tube set), characterized in that it is configured to be arranged in a heat removal section of a fluidized bed reactor, the heat removal section being disposed in a fluidized bed layer of the fluidized bed reactor, the heat removal tube set comprising:

at least one first heat removal tube, which comprises n1 (2 < n1 < 30, preferably 2 < n1 < 20, and more preferably 2 < n1 < 10) straight pipes a extending in parallel to the central axis of the fluidized bed reactor and n1-1 connecting fittings for connecting two adjacent straight pipes a in series and providing a fluid communication between them; and

at least one second heat removal tube, which comprises n2 (2 < n2 < 30, preferably 2 < n2 < 20, more preferably 2 < n2 < 10) straight pipes b extending in parallel to the central axis of the fluidized bed reactor and n2-1 connecting fittings for connecting two adjacent straight pipes b in series and providing a fluid communication between them,

wherein, in a cross section obtained by transecting along a direction perpendicular to the central axis of the fluidized bed reactor at any position of the heat removal section (where the length of the heat removal section in the direction of the central axis of the fluidized bed reactor is set as L (in m), preferably within the entire region of the length L of the heat removal section, more preferably within the region from 49% L above to 49% L below the central point of the reaction heat removal section, more preferably within the region from 45% L above to 38% L below the central point of the reaction heat removal section, more preferably within the region from 40% L above to 8% L below the central point of the reaction heat removal section), more than 50% (preferably 60% or more, more preferably 70% or more) of the total straight pipes a of the first heat removal tube are within the central part of the cross section of the heat removal section of the fluidized bed reactor, and less than 50% (preferably 40% or less, more preferably 30% or less) of the total straight pipes b of the second heat removal tube are within the central part of the cross section, and

the ratio of the total circumference Lb of the outer contours of all of the straight pipes b of the second heat removal tube (where there are a plurality of second heat removal tubes, all of the second heat removal tubes) on the cross section to the total circumference La of the outer contours of all of the straight pipes a of the first heat removal tube (where there are a plurality of first heat removal tubes, all of the first heat removal tubes) on the cross section is greater than 1 and less than 1.25 (preferably greater than 1 and less than or equal to 1.15 or 1.01-1.10) or greater than 1 and less than or equal to 1.12.

2. The heat removal tube set as described in any of the preceding or subsequent aspects, characterized in that, |n1-n2| < 5 (preferably |n1-n2| < 3) is satisfied.

3. The heat removal tube set as described in any of the preceding or subsequent aspects, characterized in that, where the radius of the cross section is set as R (in m), the central part is the region of 3/4R (preferably 2/3R, more preferably 1/2R, and even more preferably 1/3R) to the center of the cross section.

4. The heat removal tube set as described in any of the preceding or subsequent aspects, wherein the ratio of the outer diameter (in mm) of the straight pipe a to the outer diameter (in mm) of the straight pipe b is 1 to 1.6, preferably 1 to 1.4; alternatively, the ratio of the outer diameter (in mm) of the straight pipe b to the outer diameter (in mm) of the straight pipe a is 1 to 1.6, preferably 1 to 1.4.

5. The heat removal tube set as described in any of the preceding or subsequent aspects, characterized in that the outer diameters of the straight pipes a are respectively and independently 80-180 mm, preferably 90-170 mm, the lengths of the straight pipes a are respectively and independently 4-13 m, preferably 5-12.0 m, the distance between two adjacent straight pipes a is 100-700 mm, preferably 150-500 mm, and/or the outer diameters of the straight pipes b are respectively and independently 80-180 mm, preferably 90-170 mm, the lengths of the straight pipes b are respectively and independently 4-13 m, preferably 5-12.0 m, the distance between two adjacent straight pipes b is 100-700 mm, preferably 150-500 mm, and/or the total circumference of the outer contour of one first heat removal tube is 0.5-17 m, preferably 2.5-11.3 m, and/or the total circumference of the outer contour of one second heat removal tube is 0.5-17 m, preferably 2.5-11.3 m.

6. The heat removal tube set as described in any of the preceding or subsequent aspects, characterized in that the length L of the heat removal section is 4-12.5 m, preferably 5.5-11.5 m, and/or the radius R is 5-29 m, preferably 7-20 m, and/or the number of the first heat removal tubes is 1-4 or 1, and/or the number of the second heat removal tubes is 1-4 or 1, and/or the heat removal tube set comprises at least one pair (preferably 1-20 pairs, more preferably 2-10 pairs or 2-5 pairs) of heat removal tubes, and each pair of the heat removal tubes is composed of the at least one first heat removal tube and the at least one second heat removal tube.

7. The heat removal tube set as described in any of the preceding or subsequent aspects, characterized in that under the same operating conditions of the fluidized bed reactor, the difference (absolute value) between the magnitude of the modulation of the reaction temperature of the fluidized bed reactor by the first heat removal tube (where there are a plurality of first heat removal tubes, all of the first heat removal tubes) and the magnitude of the modulation of the reaction temperature of the fluidized bed reactor by the second heat removal tube (where there are a plurality of second heat removal tubes, all of the second heat removal tubes) is 0.1 to 1 °C, preferably 0.1 to 0.5 °C, and/or, where there are a plurality of the first heat removal tubes, the cooling water inlets of at least 2 (preferably all) of the first heat removal tubes are merged into a cooling water inlet header in the heat removal section, and/or, where there are a plurality of the first heat removal tubes, the cooling water outlets of at least 2 (preferably all) of the first heat removal tubes are merged into a cooling water outlet header in the heat removal section, and/or, where there are a plurality of second heat removal tubes, the cooling water inlets of at least 2 (preferably all) of the second heat removal tubes are merged into a cooling water inlet header in the heat removal section, and/or, where there are a plurality of second heat removal tubes, the cooling water outlets of at least 2 (preferably all) of the second heat removal tubes are merged into a cooling water outlet header in the heat removal section.

8. A fluidized bed reactor, characterized in that it comprises a head, a dilute phase zone, a heat removal section, a pre-reaction section and a cone from top to bottom in sequence, wherein a heat removal tube set as described in any of the preceding or subsequent aspects is arranged in the heat removal section.

9. A method for controlling the temperature of the fluidized bed reactor as described in any of the preceding or subsequent aspects, comprising the step of switching the first heat removal tube (where there are a plurality of first heat removal tubes, all of the first heat removal tubes) to the second heat removal tube (where there are a plurality of second heat removal tubes, all of the second heat removal tubes) during the reaction process, so as to maintain the reaction temperature of the fluidized bed reactor substantially constant (preferably, the absolute value of the amplitude of variation is 0.1-1 °C, preferably 0.1-0.5 °C).

10. A method for producing an unsaturated nitrile, comprising the step of subjecting an olefin (such as propylene) to an ammoxidation reaction in a fluidized bed reactor as described in any of the preceding or subsequent aspects to obtain an unsaturated nitrile (such as acrylonitrile).

11. A method for producing an unsaturated nitrile, comprising the step of subjecting an olefin (such as propylene) to an ammoxidation reaction in a fluidized-bed reactor to obtain an unsaturated nitrile (such as acrylonitrile), wherein the temperature of the fluidized-bed reactor is controlled by the method for controlling the temperature as described in any of the preceding or subsequent aspects.

12. The method as described in any of the preceding or subsequent aspects, wherein the molar ratio of propylene/ammonia/air (calculated as molecular oxygen) is 1: 1.1-1.3: 1.8-2.0, the reaction temperature is 420-440 °C, the reaction pressure (gauge pressure) is 0.03-0.14 MPa, and the weight hourly space velocity of the catalyst is 0.04-0.15 $h^{-1}$.

## Brief Description of the Drawings

Fig. 1 is a schematic front view of an existing fluidized bed reactor.
Fig. 2 is a schematic top view of an existing reaction heat removal tube set for fluidized bed.
Fig. 3 is a schematic view of a heat removal tube set according to the present application.
Figs. 4 and 5 are schematic views of the heat removal tube header of the present application.

Description of the reference numerals:

1: wall of fluidized bed reactor
2: heat removal tube of fluidized bed reactor
3: cooling water inlet of heat removal tube
4: cooling water outlet of heat removal tube
5: straight pipe of heat removal tube
6: connecting fitting of heat removal tube
7: distribution plate for oxygen-containing gas
8: propylene-ammonia distributor
9: high-efficiency cyclone separator
10: first heat removal tube
20: second heat removal tube

## Technical effects

By using the heat removal tube set according to the present application, difference between the heat quantity removed by different heat removal tubes at different positions can be compensated, so that the reaction temperature may be maintained to be substantially constant by switching the first heat removal tube and the second heat removal tube coordinatively in a paired manner.

By using the heat removal tube set and the fluidized bed reactor according to the present application, the operation stability of the equipment can be improved.

**Detailed Description of the Invention**

The present application will be illustrated in detail hereinbelow with reference to embodiments thereof, but it should be noted that the scope of the present application is not limited by those embodiments, but is defined by the appended claims.

All publications, patent applications, patents, and other references cited herein are incorporated by reference in their entirety. Unless defined otherwise, all technical and scientific terms used herein have the same meaning as commonly understood by those skilled in the art. In case of conflict, the contents described herein, including definitions, should prevail.

Where a material, substance, method, step, device, component, or the like is described herein as "commonly known to those skilled in the art", "prior art" or the like, it is to be understood that said material, substance, method, step, device and component cover not only those conventionally used in the art at the time of filing the present application, but also those not commonly used at present but will become commonly known in the art to be suitable for a similar purpose.

In the context of the present application, the term "substantially" means that a deviation acceptable or considered reasonable by those skilled in the art may be present, such as a deviation within $\pm 10\%$, within $\pm 5\%$, within $\pm 1\%$, within $\pm 0.5\%$ or within $\pm 0.1\%$.

In the context of the present application, unless specifically stated otherwise, all percentages, parts, ratios, etc. are expressed by weight and all pressures given are gauge pressures.

In the context of the present application, any two or more embodiments of the present application may be arbitrarily combined, and the resulting technical solution forms a part of the initial disclosure of the present application and falls within the scope of the present application.

According to an embodiment, the present application relates to a heat removal tube set, particularly a heat removal water tube set. According to the present application, a "heat removal tube set" and "heat removal tube" may be used to remove excess heat from a reactor in which an exothermic reaction (or some exothermic stages of the reaction) is conducted, to maintain the reaction within a certain temperature range. Examples of the reactor include a fluidized bed reactor, and more particularly, a fluidized bed reactor for producing acrylonitrile.

According to an embodiment of the present application, the heat removal tube set is configured to be arranged in a heat removal section of a fluidized bed reactor. Obviously, the heat removal tube is also configured to be arranged in the heat removal section of the fluidized bed reactor. Specifically, the straight pipes of the heat removal tubes are substantially positioned in the dense-phase area of the fluidized bed reactor and are used for timely removing reaction heat out of the system and maintaining a stable operation of the system. For this reason, in the context of the present specification, the "heat removal section" refers to the region of the fluidized bed reactor in which the heat removal tubes are disposed, more particularly the region of the fluidized bed reactor in which the straight pipes of the heat removal tubes are disposed, more particularly the region in the dense phase region of the fluidized bed reactor in which the straight pipes of the heat removal tubes are disposed.

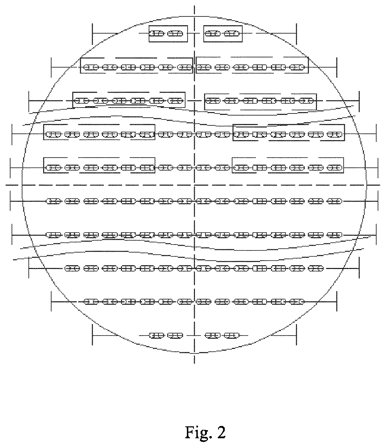

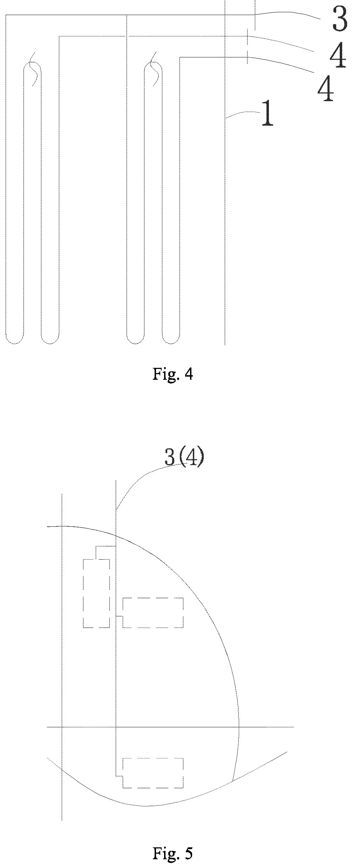

In prior arts, the heat removal tube sets in the heat removal section are typically arranged in the manner shown in Fig. 2, i.e., the heat removal tubes are arranged in a straight line. On the other hand, as shown in Fig. 1, other internal components such as dipleg of cyclone 9 are also included in the heat removal section of the fluidized bed reactor. Typically, the heat removal tubes comprise a cooling water inlet, straight pipes and a cooling water outlet, and connecting fittings for connecting these pipes in a fluid communication manner. As shown in Fig. 1 or Fig. 4, each heat removal tube comprises a cooling water inlet 3, a cooling water outlet 4, a plurality of straight pipes, and a connecting fitting for connecting any two adjacent straight pipes thereof in series and providing a fluid communication between them.

According to an embodiment of the present application, the heat removal tube set comprises at least one first heat removal tube. Here, the first heat removal tube comprises n1 ($2 < n1 < 30$, preferably $2 < n1 < 20$, more preferably $2 < n1 < 10$) straight pipes a extending in parallel to the central axis of the fluidized bed reactor and n1-1 connecting fittings for connecting two adjacent straight pipes a in series and providing a fluid communication between them.

According to an embodiment of the present application, the number of the first heat removal tubes is 1 to 4 or 1.

According to an embodiment of the present application, the heat removal tube set comprises at least one second heat removal tube, and the second heat removal tube comprises n2 ($2 < n2 < 30$, preferably $2 < n2 < 20$, more preferably $2 < n2 < 10$) straight pipes b extending in parallel to the central axis of the fluidized bed reactor and n2-1 connecting fittings for connecting two adjacent straight pipes b in series and providing a fluid communication between them.

According to an embodiment of the present application, the number of the second heat removal tubes is 1-4 or 1.

According to an embodiment of the present application, the heat removal tube set comprises at least one pair (preferably 1 to 20 pairs, more preferably 2 to 10 pairs or 2 to 5 pairs) of heat removal tubes, and each pair of the heat removal tubes is composed of the at least one first heat removal tube and the at least one second heat removal tube. In other words, according to the embodiment of the present application, the heat removal tube set comprises at least one (preferably 1 to 20, more preferably 2 to 10 or 2 to 5) heat removal tube pairs, and each of the heat removal tube pairs is composed of the at least one first heat removal tube and the at least one second heat removal tube. In the following context of the present specification, unless otherwise specified, the first heat removal tube and the second heat removal tube both refer to the first heat removal tube and the second heat removal tube in the same heat removal tube pair. The present application is not intended to pose any limitation, in different heat removal tube pairs, to the relationship between a first heat removal tube in a heat removal tube pair and a second heat removal tube in another heat removal tube pair, or the relationship between a first heat removal tube in a heat removal tube pair and a first heat removal tube in another heat removal tube pair, or the relationship between a second heat removal tube in a heat removal tube pair and a first heat removal tube in another heat removal tube pair, or the relationship between a second heat removal tube in a heat removal tube pair and a second heat removal tube in another heat removal tube pair.

According to the present application, the at least one first heat removal tube and the at least one second heat removal tube do not work at the same time, but work in a switchable manner according to the need of production operation, that is, when the at least one first heat removal tube is in a heat removal operation state, the at least one second heat removal tube is in an idle state, and for the first heat removal tube and the second heat removal tube, when a valve between the external cooling coil of the reactor and the first heat removal tube is closed, the circulation of coolant in the first heat removal tube is cut off, so that the first heat removal tube in heat removal operation is changed to an idle state, and meanwhile, a valve between the external cooling coil of the reactor and the second heat removal tube is opened, so that the circulation of coolant in the second heat removal tube is started, and the second heat removal tube in an idle state is changed to a heat removal operation state; conversely, the first heat removal tube and the second heat removal tube can also be switched reversely, when the valve between the external cooling coil of the reactor and the first heat removal tube is opened, the circulation of coolant in the first heat removal tube is started, the first heat removal tube in an idle state is changed to a heat removal operation state, and meanwhile, the valve between the external cooling coil of the reactor and the second heat removal tube is closed, the circulation of coolant in the second heat removal tube is cut off, so that the second heat removal tube in heat removal operation is changed to an idle state. Obviously, as mentioned above, the at least one first heat removal tube and the at least one second heat removal tube are heat removal tubes in the same heat removal tube pair.

According to the present application, under the same operation conditions of the fluidized bed reactor, the difference (absolute value) between the magnitude of the modulation of the reaction temperature of the fluidized bed reactor by the first heat removal tube (where there are a plurality of first heat removal tubes, all of the first heat removal tubes) and the magnitude of the modulation of the reaction temperature of the fluidized bed reactor by the second heat removal tube (where there are a plurality of second heat removal tubes, all of the second heat removal tubes) is 0.1-1 °C, preferably 0.1-0.5 °C. By such a configuration, the present application can realize a substantially constant control of the reaction temperature when heat removal tubes in the same heat removal tube pair are switched.

According to an embodiment of the present application, where a length of the heat removal section along the central axis of the fluidized bed reactor is set as L (in m), a cross section of the heat removal section is obtained by transecting along a direction perpendicular to the central axis of the fluidized bed reactor at a position within the entire region of the length L of the heat removal section. Here, the cross section of the heat removal section refers to the cross section of the inner contour of the fluidized bed reactor at the heat removal section. As the region, it is preferably within the region from 49% L above to 49% L below the center point of the reaction heat removal section, more preferably within the region from 45% L above to 38% L below the center point of the reaction heat removal section, and still more preferably within the region from 40% L above to 8% L below the center point of the reaction heat removal section. For example, the length L of the heat removal section is 4-12.5 m, preferably 5.5-11.5 m.

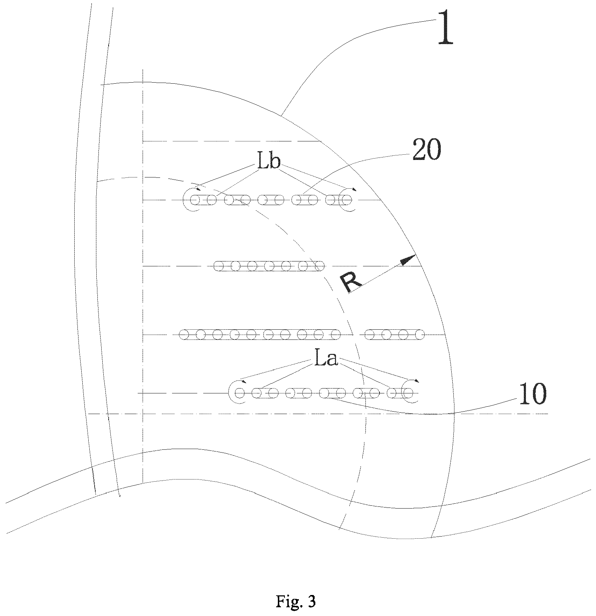

According to an embodiment of the present application, as illustrated in Fig. 3, in a cross section of the heat removal section, more than 50% (preferably 60% or more, more preferably 70% or more) of the total straight pipes a of the first heat removal tube are within a central part of a cross section of the heat removal section of the fluidized bed reactor, and less than 50% (preferably 40% or less, more preferably 30% or less) of the total straight pipes b of the second heat removal tube are within a central part of the cross section. For example, when the first heat removal tube 10 has 8 straight pipes, 5 or more straight pipes are within the central part of the cross section, or when the first heat removal tube 10 has 7 straight pipes, 4 or more straight pipes are within the central part of the cross section. On the other hand, for example, when the second heat removal tube 20 has 6 straight pipes, 2 or less straight pipes are within the central part of the cross section, or when the second heat removal tube 20 has 5 straight pipes, 2 or less straight pipes are within the central part of the cross section.

According to the present application, where the radius of the circular cross section of the heat removal section of the fluidized bed reactor shown in Fig. 3 is set as R (in m), the central part of the cross section refers to a region within a certain distance from the center of the circular cross section (i.e., a region within the dotted line in Fig. 3), and the peripheral region of the cross section refers to the region out of the central part (i.e., a region from the dotted line in Fig. 3 to the reactor wall 1). According to an embodiment of the present application, the central part of the cross section refers to a circular region within a distance of 3/4R from the center of the cross section, preferably within a distance of 2/3R from the center of the cross section, more preferably within a distance of 1/2R from the center of the cross section, and even more preferably within a distance of 1/3R from the center of the cross section. For example, the radius R is from 5 to 29 m, preferably from 7 to 20 m.

According to an embodiment of the present application, a ratio between the total circumference Lb of the outer contours of all of the straight pipes b of the second heat removal tube (where there are a plurality of second heat removal tubes, all of the second heat removal tubes) on the cross section and the total circumference La of the outer contours of all of the straight pipes a of the first heat removal tube (where there are a plurality of first heat removal tubes, all of the first heat removal tubes) on the cross section is greater than 1 and less than 1.25, preferably greater than 1 and less than or equal to 1.15, or 1.01 to 1.10, or greater than 1 and less than or equal to 1.12. When Lb/La is greater than 1.25, during the switching of the first heat removal tube and the second heat removal tube, the increase of the heat removal capacity caused by the increase of the heat removal area is higher than the variation of the heat removal capacity caused by the difference between the heat conduction capacities of different heat removal tubes, namely the heat removal tube set cannot meet the requirement of equivalent heat removal capacity, which leads to a deviation of the reaction temperature from the pre-determined temperature; similarly, when Lb/La is less than 1, the requirement of equivalent heat removal capability cannot be met when a switch of heat removal tubes of a heat removal tube set pair is conducted.

According to an embodiment of the present application, the total circumference of the outer contour of one first heat removal tube is 0.5-17 m, preferably 2.5-11.3 m.

According to an embodiment of the present application, the total circumference of the outer contour of one second heat removal tube is 0.5-17 m, preferably 2.5-11.3 m.

According to an embodiment of the present application, where there are a plurality of first heat removal tubes, the cooling water inlets of at least 2 (preferably all) of the first heat removal tubes are merged into a cooling water inlet header in the heat removal section. In other words, a plurality of the heat removal tubes (referred to as branch pipes) share one cooling water inlet. According to the present application, the cooling water inlet header is in fluid communication with an external cooling water supply source through the wall of the fluidized bed reactor, whereby the cooling water is supplied to respective branch pipes through the cooling water inlet header.

According to an embodiment of the present application, where there are a plurality of first heat removal tubes, the cooling water outlets of at least 2 (preferably all) of the first heat removal tubes are merged into a cooling water outlet header in the heat removal section. In other words, a plurality of the heat removal tubes (referred to as branch pipes) share one cooling water outlet. According to the present application, the cooling water outlet header is in fluid communication with an external cooling water receiving means through the wall of the fluidized bed reactor, whereby cooling water (typically also containing steam) after heat removal is delivered from respective branch pipes to the external environment through the cooling water outlet header.

According to an embodiment of the present application, when there are a plurality of second heat removal tubes, the cooling water inlets of at least 2 (preferably all) of the second heat removal tubes are merged into a cooling water inlet header in the heat removal section. In other words, a plurality of the heat removal tubes (referred to as branch pipes) share one cooling water inlet. According to the present application, the cooling water inlet header is in fluid communication with an external cooling water supply source through the wall of the fluidized bed reactor, whereby the cooling water is supplied to respective branch pipes through the cooling water inlet header.

According to an embodiment of the present application, where there are a plurality of second heat removal tubes, the cooling water outlets of at least 2 (preferably all) of the second heat removal tubes are merged into a cooling water outlet header in the heat removal section. In other words, a plurality of the heat removal tubes (referred to as branch pipes) share one cooling water outlet. According to the present application, the cooling water outlet header is in fluid communication with an external cooling water receiving means through the wall of the fluidized bed reactor, whereby cooling water (typically also containing steam) after heat removal is delivered from respective branch pipes to the external environment through the cooling water outlet header.

Figs. 4 and 5 are schematic views of the arrangement of the heat removal tube header of the present application. As can be seen from the figure, the cooling water inlets/outlets of a plurality of heat removal tubes are merged into a header.

According to an embodiment of the present application, a ratio of a cross-sectional area of the header (such as the cooling water inlet header or the cooling water outlet header) to a sum of cross-sectional areas of the plurality of branch pipes corresponding thereto (generally, based on cooling water inlets or cooling water outlets of the plurality of branch pipes) is 0.5 to 1, preferably 0.55 to 0.95, and more preferably 0.6 to 0.9.

According to an embodiment of the present application, $|n1-n2| < 5$ (preferably $|n1-n2| < 3$) is satisfied. The circumference of the outer contour of one heat removal tube is the sum of the circumferences of the outer contours of n heat removal straight pipes thereof or the circumference of the outer contour can be directly expressed by $n*3.14*D$ (D is the average outer diameter of the heat removal straight pipes), and typically the larger the number of the straight pipes is, the longer the circumference of the outer contour is, and the stronger the heat removal capability is in the operation process of the equipment; therefore, for the heat removal tube set, $|n1-n2|$ is too large, which means that the difference between the circumferences of the outer contours of the first heat removal tube and the second heat removal tube is larger, which may easily cause a shift of the reaction temperature during the switching of the heat removal tube pair, or different outer diameter may also be used for these tubes, |n1-n2| is too large, the difference between the outer diameters of the first heat removal tube and the second heat removal tube is also large, which obviously is unreasonable and uneconomical.

According to an embodiment of the present application, the ratio of the outer diameter (in mm) of the straight pipe a to the outer diameter (in mm) of the straight pipe b is 1 to 1.6, preferably 1 to 1.4.

According to an embodiment of the present application, the ratio of the outer diameter (in mm) of the straight pipe b to the outer diameter (in mm) of the straight pipe a is 1 to 1.6, preferably 1 to 1.4.

According to an embodiment of the present application, the outer diameters of the straight pipes a are respectively 80-180 mm, preferably 90-170 mm, the lengths of the straight pipes a can be the same or different, the lengths of the straight pipes a are respectively 4-13 m, preferably 5-12.0 m, and the spacing between two adjacent straight pipes a is 100-700 mm, preferably 150-500 mm.

According to an embodiment of the present application, the outer diameters of the straight pipes b are respectively 80-180 mm, preferably 90-170 mm, the lengths of the straight pipes b can be the same or different, the lengths of the straight pipes b are respectively 4-13 m, preferably 5-12.0 m, and the spacing between two adjacent straight pipes b is 100-700 mm, preferably 150-500 mm.

According to an embodiment, the present application also relates to a heat removal tube set, characterized in that the heat removal tube set is arranged in a heat removal section of a fluidized bed reactor, the heat removal section being disposed in a fluidized bed layer of the fluidized bed reactor, and the heat removal tube set comprises: at least one first heat removal tube, which comprises $n1$ ($2 < n1 < 30$, preferably $2 < n1 < 20$, more preferably $2 < n1 < 10$) straight pipes a extending in parallel to the central axis of the fluidized bed reactor and $n1-1$ connecting fittings for connecting the $n1$ straight pipes in series and providing a fluid communication between them; and at least one second heat removal tube, which comprises $n2$ ($2 < n1 < 30$, preferably $2 < n1 < 20$, more preferably $2 < n1 < 10$) straight pipes b extending in parallel to the central axis of the fluidized bed reactor and $n2-1$ connecting fittings for connecting the $n2$ straight pipes in series and provide a fluid communication between them, wherein, in a cross section obtained by transecting along a direction perpendicular to the central axis of the fluidized bed reactor at any position of the heat removal section, more than 50% (preferably 60% or more, more preferably 70% or more) of the total straight pipes a of the first heat removal tube are within the central part of the cross section of the heat removal section of the fluidized bed reactor, and less than 50% (preferably 40% or less, more preferably 30% or less) of the total straight pipes b of the second heat removal tube are within the central part of the cross section, and the ratio of the total circumference $Lb$ of the outer contours of all of the straight pipes b of the second heat removal tube to the total circumference $La$ of the outer contours of all of the straight pipes a of the first heat removal tube is greater than 1 and less than or equal to 1.1 or greater than 1 and less than or equal to 1.12.

According to an embodiment, the present application also relates to a fluidized bed reactor. The fluidized bed reactor sequentially comprises a head, a dilute phase zone, a heat removal section, a pre-reaction section and a cone from top to bottom, wherein a heat removal tube set as described in any of the preceding or subsequent aspects of the present application is arranged in the heat removal section.

According to an embodiment, the present application also relates to a method for controlling the temperature of a fluidized bed reactor. Here, the fluidized bed reactor is preferably the fluidized bed reactor described hereinabove.

According to an embodiment of the present application, the control method comprises switching the first heat removal tube (when a plurality of first heat removal tubes are present, they are combined together) to the second heat removal tube (when a plurality of second heat removal tubes are present, they are combined together) during the reaction process to maintain the reaction temperature of the fluidized bed reactor substantially constant, preferably with an absolute value of the amplitude of variation of 0.1 to 1 °C, preferably 0.1 to 0.5 °C.

According to an embodiment, the present application also relates to a method for producing an unsaturated nitrile, comprising the step of subjecting an olefin (such as propylene) to an ammoxidation reaction in a fluidized-bed reactor to obtain an unsaturated nitrile (such as acrylonitrile). Here, the fluidized bed reactor is preferably the fluidized bed reactor described hereinabove.

According to an embodiment, the present application also relates to a method for producing an unsaturated nitrile, comprising the step of subjecting an olefin (such as propylene) to an ammoxidation reaction in a fluidized-bed reactor to obtain an unsaturated nitrile (such as acrylonitrile). Here, the temperature of the fluidized bed reactor may be controlled according to the method for controlling the temperature as described hereinabove to maintain the reaction temperature of the fluidized bed reactor substantially constant.

According to an embodiment of the present application, the ammoxidation reaction may be performed in any manner and by any method conventionally known in the art, and such information is known to those skilled in the art and will not be described herein in detail. However, specific examples of the conditions for the ammoxidation reaction include a molar ratio of propylene to ammonia to air (calculated as molecular oxygen) of typically 1: 1.1-1.3: 1.8-2.0, a reaction temperature of typically 420-440 °C, a reaction pressure (gauge pressure) of typically 0.03-0.14 MPa, and a weight hourly space velocity of the catalyst of typically 0.04-0.15 h$^{-1}$.

**Examples**

The present application will be further illustrated in detail with reference to the following examples and comparative examples, but the present application is not limited to those examples.

In the following examples and comparative examples, the acrylonitrile yield and the propylene conversion can be calculated according to the following equations:

$$\text{Yield of acrylonitrile: } AN\% = C_{AN}/\Sigma C * 100$$

$$\text{Conversion of propylene: } Cc_3\% = (1-Cc_{3out}/\ Cc_{3in}) * 100$$

wherein:

$C_{AN}$: molar amount (mol) of carbon contained in AN in the gas at the outlet of the reactor
$\Sigma C$: total molar amount (mol) of carbon in the gas at the outlet of the reactor
$Cc_{3out}$: molar amount (mol) of carbon contained in $C_3$ in the gas at the outlet of the reactor
$Cc_{3in}$: molar amount (mol) of carbon contained in $C_3$ in the gas at the inlet of the reactor.

Example 1

The fluidized bed reactor had a diameter of 9 meters, 180 tons of acrylonitrile catalysts of SANC series of Sinopec Shanghai Research Institute of Petrochemical Technology Co., Ltd. were filled therein, 584 straight pipes with the same height were arranged in the reactor, which were divided into 56 heat removal tubes. The heat removal tubes were formed into 4 groups of heat removal tube pairs, wherein one heat removal tube in each group of heat removal tube pairs was a second heat removal tube formed by connecting 7 straight pipes b in series, and 5 straight pipes b of the second heat removal tube were positioned outside the region from 2/3R to the center of the cross section of the heat removal section of the reactor. The other heat removal tube was a first heat removal tube formed by connecting 8 straight pipes a in series, and 5 straight pipes b of the first heat removal tube were positioned within the region from 2/3R to the center of the cross section of the heat removal section of the reactor. The ratio of the outer diameter of the branch pipe b to the outer diameter of the branch pipe a was 1.3, and the ratio of the total circumference of the outer contours of all of the straight pipes b constituting the second heat removal tube to the total circumference of the outer contours of the straight pipes a constituting the first heat removal tube was 1.12.

The feed rate of propylene was 11800 NM$^3$/h, the reaction temperature was 430 °C, the reaction pressure was 0.04 MPa and the ratio of propylene: ammonia: air was 1: 1.2: and 9.6, the amplitude of fluctuation of the reaction temperature was 0.8 °C by switching between the first heat removal tube and the second heat removal tube of the heat removal tube set.

Example 2

The fluidized bed reactor had a diameter of 9 meters, 180 tons of acrylonitrile catalysts of SANC series of Sinopec Shanghai Research Institute of Petrochemical Technology Co., Ltd. were filled therein, 584 straight pipes with the same height were arranged in the reactor, which were divided into 56 heat removal tubes. The heat removal tubes were formed into 5 groups of heat removal tube pairs, wherein one heat removal tube in each group of heat removal tube pairs was a second heat removal tube formed by connecting 10 straight pipes b in series, and 8 straight pipes b of the second heat removal tube were positioned outside the region from 2/3R to the center of the cross section of the heat removal section of the reactor. The other heat removal tube was a first heat removal tube formed by connecting 12 straight pipes a in series, and 6 straight pipes a of the first heat removal tube were positioned within the region from 2/3R to the center of the cross section of the heat removal section of the reactor. The ratio of the outer diameter of the branch pipe b to the outer diameter of the branch pipe a was 1.25, and the ratio of the total circumference of the outer contours of all of the straight pipes constituting the second heat removal tube to the total circumference of the outer contours of the straight pipes constituting the first heat removal tube was 1.05.

The feed rate of propylene was 11800 NM$^3$/h, the reaction temperature was 430 °C, the reaction pressure was 0.04 MPa and the ratio of propylene: ammonia: air was 1: 1.2: and 9.6, the amplitude of fluctuation of the reaction temperature was 0.4 °C by switching between the first heat removal tube and the second heat removal tube of the heat removal tube set.

Example 3

The fluidized bed reactor had a diameter of 9 meters, 180 tons of acrylonitrile catalysts of SANC series of Sinopec Shanghai Research Institute of Petrochemical Technology Co., Ltd. were filled therein, 584 straight pipes with the same height were arranged in the reactor, which were divided into 56 heat removal tubes. The heat removal tubes were formed into 4 groups of heat removal tube pairs, wherein one heat removal tube was a first heat removal tube formed by connecting 10 straight pipes a in series, and 8 straight pipes a of the first heat removal tube were positioned within the region from 2/3R to the center of the cross section of the heat removal section of the reactor; the other heat removal tube was a second heat removal tube formed by 6 straight pipes b connected in series and 7 straight pipes b connected in series, and 8 straight pipes b of the second heat removal tube were positioned outside the region from 2/3R to the center of the cross section of the heat removal section of the reactor. The ratio of the outer diameter of the branch pipe a to the outer diameter of the branch pipe b was 1.28, and the ratio of the total circumference of the outer contours of all of the straight pipes constituting the second heat removal tube to the total circumference of the outer contours of the straight pipes constituting the first heat removal tube was 1.02.

The feed rate of propylene was 11800 NM$^3$/h, the reaction temperature was 430 °C, the reaction pressure was 0.04 MPa and the ratio of propylene: ammonia: air was 1: 1.2: and 9.6, the amplitude of fluctuation of the reaction temperature was 0.3 °C by switching between the first heat removal tube and the second heat removal tube of the heat removal tube set.

Example 4

The fluidized bed reactor had a diameter of 9 meters, 180 tons of acrylonitrile catalysts of SANC series of Sinopec Shanghai Research Institute of Petrochemical Technology Co., Ltd. were filled therein, 732 straight pipes with the same height were arranged in the reactor, which were divided into 70 heat removal tubes. The heat removal tubes were formed into 5 groups of heat removal tube pairs, wherein in 2 groups of heat removal tube pairs, one heat removal tube was a second heat removal tube formed by 10 straight pipes b connected in series and 9 straight pipes b connected in series, and 15 straight pipes a in the second heat removal tube were positioned outside the region from 2/3R to the center of the cross section of the heat removal section of the reactor; the other heat removal tube was a first heat removal tube formed by 10 straight pipes a connected in series and 8 straight pipes a connected in series, and 15 straight pipes a of the first heat removal tube were positioned within the region from 2/3R to the center of the cross section of the heat removal section of the reactor. The ratio of the outer diameter of the branch pipe a to the outer diameter of the branch pipe b was 1, and the ratio of the total circumference of the outer contours of all of the straight pipes constituting the second heat removal tube to the total circumference of the outer contours of the straight pipes constituting the first heat removal tube was 1.05.

The feed rate of propylene was 11800 NM$^3$/h, the reaction temperature was 430 °C, the reaction pressure was 0.04 MPa and the ratio of propylene: ammonia: air was 1: 1.2: and 9.6, the amplitude of fluctuation of the reaction temperature was 0.3 °C by switching between the first heat removal tube and the second heat removal tube of the heat removal tube set.

Example 5

The fluidized bed reactor had a diameter of 9 meters, 180 tons of acrylonitrile catalysts of SANC series of Sinopec Shanghai Research Institute of Petrochemical Technology Co., Ltd. were filled therein, 732 straight pipes with the same height were arranged in the reactor, which were divided into 70 heat removal tubes. The heat removal tubes were formed into 5 groups of heat removal tube pairs, wherein in one group of heat removal tube pairs, one heat removal tube was a second heat removal tube formed by 10 straight pipes b connected in series and 12 straight pipes b connected in series, and 18 straight pipes a of the second heat removal tube were positioned outside the region from 2/3R to the center of the cross section of the heat removal section of the reactor; the other heat removal tube was a first heat removal tube formed by 8 straight pipes a connected in series and 8 straight pipes a connected in series, and 15 straight pipes a of the first heat removal tube were positioned within the region from 2/3R to the center of the cross section of the heat removal section of the reactor. The ratio of the outer diameter of the branch pipe a to the outer diameter of the branch pipe b was 1.28, and the ratio of the total circumference of the outer contours of all of the straight pipes constituting the second heat removal tube to the total circumference of the outer contours of the straight pipes constituting the first heat removal tube was 1.07.

The feed rate of propylene was 11800 NM$^3$/h, the reaction temperature was 430 °C, the reaction pressure was 0.04 MPa and the ratio of propylene: ammonia: air was 1: 1.2: and 9.6, the amplitude of fluctuation of the reaction temperature was 0.5 °C by switching between the first heat removal tube and the second heat removal tube of the heat removal tube set.

Example 6

The fluidized bed reactor had a diameter of 9 meters, 180 tons of acrylonitrile catalysts of SANC series of Sinopec Shanghai Research Institute of Petrochemical Technology Co., Ltd. were filled therein, 680 straight pipes with the same height were arranged in the reactor, which were divided into 56 heat removal tubes. The heat removal tubes were formed into 2 groups of heat removal tube pairs, wherein one heat removal tube was a second heat removal tube formed by connecting 9 straight pipes b in series, and 6 straight pipes b of the second heat removal tube were positioned outside the region from 2/3R to the center of the cross section of the heat removal section of the reactor; the other heat removal tube was a first heat removal tube formed by connecting 10 straight pipes a in series, and 8 straight pipes a of the first heat removal tube were positioned within the region from 2/3R to the center of the cross section of the heat removal section of the reactor. The ratio of the outer diameter of the branch pipe b to the outer diameter of the branch pipe a was 1.17, and the ratio of the total circumference of the outer contours of all of the straight pipes constituting the second heat removal tube to the total circumference of the outer contours of the straight pipes constituting the first heat removal tube was 1.05.

The feed rate of propylene was 11800 NM$^3$/h, the reaction temperature was 430 °C, the reaction pressure was 0.04 MPa and the ratio of propylene: ammonia: air was 1: 1.2: and 9.6, the amplitude of fluctuation of the reaction temperature was 0.4 °C by switching between the first heat removal tube and the second heat removal tube of the heat removal tube set.

Example 7

The fluidized bed reactor had a diameter of 9 meters, 180 tons of acrylonitrile catalysts of SANC series of Sinopec Shanghai Research Institute of Petrochemical Technology Co., Ltd. were filled therein, 732 straight pipes with the same height were arranged in the reactor, which were divide into 70 heat removal tubes, for 4 groups of which, 3 heat removal tube sets were connected in parallel, forming into 2 groups of heat removal tube pairs, wherein one heat removal tube was a second heat removal tube formed by 12 straight pipes b connected in series, 10 straight pipes b connected in series and 10 straight pipes b connected in series, 22 straight pipes b of the second heat removal tube were positioned outside the region from 2/3R to the center of the cross section of the heat removal section of the reactor; the other heat removal tube was a first heat removal tube formed by 12 straight pipes a connected in series, 12 straight pipes a connected in series and 7 straight pipes a connected in series, and 18 straight pipes b of the first heat removal tube were positioned within the region from 2/3R to the center of the cross section of the heat removal section of the reactor. The outer diameter of the heat removal tube was 89 mm, the ratio of the outer diameter of the branch pipe a to the outer diameter of the branch pipe b was 1, and the ratio of the total circumference of the outer contours of all of the straight pipes constituting the second heat removal tube to the total circumference of the outer contours of the straight pipes constituting the first heat removal tube was 1.03. The outer diameter of the inlet header of the heat removal tube was 140 mm, the outer diameter of the outlet header of the heat removal tube was 150 mm, the ratio of the cross sectional area of the inlet header to the sum of the cross sectional areas of the heat removal branch pipes was 0.82, and the ratio of the cross sectional area of the outlet header to the sum of the cross sectional areas of the heat removal branch pipes was 0.95.

The feed rate of propylene was 11800 NM$^3$/h, the full propylene treatment capacity at this time was 349kg propylene/m$^2$/h, the reaction temperature was 430 °C, the reaction pressure was 0.04MPa, the ratio of propylene: ammonia: air was 1: 1.2: and 9.6, the amplitude of fluctuation of the reaction temperature was 0.5 °C by switching between the first heat removal tube and the second heat removal tube of the heat removal tube set.

Comparative Example 1

The fluidized bed reactor had a diameter of 9 meters, 180 tons of acrylonitrile catalysts of SANC series of Sinopec Shanghai Research Institute of Petrochemical Technology Co., Ltd. were filled therein, 416 straight pipes with the same height were arranged in the reactor, which were divided into 44 heat removal tubes. One heat removal tube was the first heat removal tube formed by connecting 6 straight pipes a in series, and 4 straight pipes a of the first heat removal tube were positioned within the region from 2/3R to the center of the cross section of the heat removal section of the reactor. One heat removal tube was a second heat removal tube formed by 6 straight pipes b, and 4 straight pipes b of the second heat removal tube were positioned outside the region from 3/4R to the center of the cross section of the heat removal section of the reactor. The ratio of the outer diameter of the branch pipe a to the outer diameter of the branch pipe b was 1, and the ratio of the total circumference of the outer contours of all of the straight pipes a constituting the first heat removal tube to the total circumference of the outer contours of the straight pipes b constituting the second heat removal tube was 1:1.

The feed rate of propylene was 7700 NM$^3$/h, the reaction temperature was 430 °C, the reaction pressure was 0.04MPa, the ratio of propylene: ammonia: air was 1: 1.2: 9.6, the amplitude of fluctuation of the reaction temperature was 1.5 °C by switching between the first heat removal tube and the second heat removal tube of the heat removal tube set.

Comparative Example 2

The fluidized bed reactor had a diameter of 9 meters, 180 tons of acrylonitrile catalysts of SANC series of Sinopec Shanghai Research Institute of Petrochemical Technology Co., Ltd. were filled therein, 584 straight pipes with the same height were arranged in the reactor, which were divided into 56 heat removal tubes. One heat removal tube was a second heat removal tube formed by connecting 11 straight pipes b in series, and 8 straight pipes b of the second heat removal tube were positioned outside the region from 2/3R to the center of the cross section of the heat removal section of the reactor. The other heat removal tube was a first heat removal tube formed by connecting 10 straight pipes a in series, and 5 straight pipes a of the first heat removal tube were positioned within the region from 2/3R to the center of the cross section of the heat removal section of the reactor. The ratio of the outer diameter of the branch pipe a to the outer diameter of the branch pipe b was 1.28, and the ratio of the total circumference of the outer contours of all of the straight pipes constituting the second heat removal tube to the total circumference of the outer contours of the straight pipes constituting the first heat removal tube was 0.86.

The feed rate of propylene was 11800 $NM^3/h$, the reaction temperature was 430 °C, the reaction pressure was 0.04 MPa and the ratio of propylene: ammonia: air was 1: 1.2: and 9.6, the amplitude of fluctuation of the reaction temperature was 1.8 °C by switching between the first heat removal tube and the second heat removal tube of the heat removal tube set.

Comparative Example 3

The fluidized bed reactor had a diameter of 9 meters, 180 tons of acrylonitrile catalysts of SANC series of Sinopec Shanghai Research Institute of Petrochemical Technology Co., Ltd. were filled therein, 584 straight pipes with the same height were arranged in the reactor, which were divided into 56 heat removal tubes. One heat removal tube was a first heat removal tube formed by connecting 7 straight pipes a in series, and 4 straight pipes a of the first heat removal tube were positioned within the region from 2/3R to the center of the cross section of the heat removal section of the reactor. The other heat removal tube was a second heat removal tube formed by connecting 9 straight pipes b in series, and 5 straight pipes b of the second heat removal tube were positioned outside the region from 2/3R to the center of the cross section of the heat removal section of the reactor. The ratio of the outer diameter of the branch pipe b to the outer diameter of the branch pipe a was 1, and the ratio of the total circumference of the outer contours of all of the straight pipes constituting the second heat removal tube to the total circumference of the outer contours of the straight pipes constituting the first heat removal tube was 1.29.

The feed rate of propylene was 11800 $NM^3/h$, the reaction temperature was 430 °C, the reaction pressure was 0.04 MPa and the ratio of propylene: ammonia: air was 1: 1.2: and 9.6, the amplitude of fluctuation of the reaction temperature was 1.6 °C by switching between the first heat removal tube and the second heat removal tube of the heat removal tube set.

**Claims**

1.  A heat removal tube set (particularly a heat removal water tube set), **characterized in that** it is configured to be arranged in a heat removal section of a fluidized bed reactor, the heat removal section being disposed in a fluidized bed layer of the fluidized bed reactor, the heat removal tube set comprising:

    at least one first heat removal tube, which comprises n1 (2 < n1 < 30, preferably 2 < n1 < 20, and more preferably 2 < n1 < 10) straight pipes a extending in parallel to the central axis of the fluidized bed reactor and n1-1 connecting fittings for connecting two adjacent straight pipes a in series and providing a fluid communication between them; and
    at least one second heat removal tube, which comprises n2 (2 < n2 < 30, preferably 2 < n2 < 20, more preferably 2 < n2 < 10) straight pipes b extending in parallel to the central axis of the fluidized bed reactor and n2-1 connecting fittings for connecting two adjacent straight pipes b in series and providing a fluid communication between them,
    wherein, in a cross section obtained by transecting along a direction perpendicular to the central axis of the fluidized bed reactor at any position of the heat removal section (where the length of the heat removal section in the direction of the central axis of the fluidized bed reactor is set as L (in m), preferably within the entire region of the length L of the heat removal section, more preferably within the region from 49% L above to 49% L below the central point of the reaction heat removal section, more preferably within the region from 45% L above to 38% L below the central point of the reaction heat removal section, more preferably within the region from 40% L above to 8% L below the central point of the reaction heat removal section), more than 50% (preferably 60% or more, more preferably 70% or more) of the total straight pipes a of the first heat removal tube are within the central part of the cross section of the heat removal section of the fluidized bed reactor, and less than 50% (preferably 40% or less, more preferably 30% or less) of the total straight pipes b of the second heat removal tube are within the central part of the cross section, and

the ratio of the total circumference Lb of the outer contours of all of the straight pipes b of the second heat removal tube (where there are a plurality of second heat removal tubes, all of the second heat removal tubes) on the cross section to the total circumference La of the outer contours of all of the straight pipes a of the first heat removal tube (where there are a plurality of first heat removal tubes, all of the first heat removal tubes) on the cross section is greater than 1 and less than 1.25 (preferably greater than 1 and less than or equal to 1.15 or 1.01-1.10) or greater than 1 and less than or equal to 1.12.

2. The heat removal tube set according to claim 1, **characterized in that**, $|n1-n2| < 5$ (preferably $|n1-n2| < 3$) is satisfied.

3. The heat removal tube set according to claim 1, **characterized in that**, where the radius of the cross section is set as R (in m), the central part is the region of 3/4R (preferably 2/3R, more preferably 1/2R, and even more preferably 1/3R) to the center of the cross section.

4. The heat removal tube set according to claim 1, wherein the ratio of the outer diameter (in mm) of the straight pipe a to the outer diameter (in mm) of the straight pipe b is 1 to 1.6, preferably 1 to 1.4; alternatively, the ratio of the outer diameter (in mm) of the straight pipe b to the outer diameter (in mm) of the straight pipe a is 1 to 1.6, preferably 1 to 1.4.

5. The heat removal tube set according to claim 1, **characterized in that** the outer diameters of the straight pipes a are respectively and independently 80-180 mm, preferably 90-170 mm, the lengths of the straight pipes a are respectively and independently 4-13 m, preferably 5-12.0 m, the distance between two adjacent straight pipes a is 100-700 mm, preferably 150-500 mm, and/or the outer diameters of the straight pipes b are respectively and independently 80-180 mm, preferably 90-170 mm, the lengths of the straight pipes b are respectively and independently 4-13 m, preferably 5-12.0 m, the distance between two adjacent straight pipes b is 100-700 mm, preferably 150-500 mm, and/or the total circumference of the outer contour of one first heat removal tube is 0.5-17 m, preferably 2.5-11.3 m, and/or the total circumference of the outer contour of one second heat removal tube is 0.5-17 m, preferably 2.5-11.3 m.

6. The heat removal tube set according to claim 1, **characterized in that** the length L of the heat removal section is 4-12.5 m, preferably 5.5-11.5 m, and/or the radius R is 5-29 m, preferably 7-20 m, and/or the number of the first heat removal tubes is 1-4 or 1, and/or the number of the second heat removal tubes is 1-4 or 1, and/or the heat removal tube set comprises at least one pair (preferably 1-20 pairs, more preferably 2-10 pairs or 2-5 pairs) of heat removal tubes, and each pair of the heat removal tubes is composed of the at least one first heat removal tube and the at least one second heat removal tube.

7. The heat removal tube set according to claim 1, **characterized in that** under the same operating conditions of the fluidized bed reactor, the difference (absolute value) between the magnitude of the modulation of the reaction temperature of the fluidized bed reactor by the first heat removal tube (where there are a plurality of first heat removal tubes, all of the first heat removal tubes) and the magnitude of the modulation of the reaction temperature of the fluidized bed reactor by the second heat removal tube (where there are a plurality of second heat removal tubes, all of the second heat removal tubes) is 0.1 to 1 °C, preferably 0.1 to 0.5 °C, and/or, where there are a plurality of the first heat removal tubes, the cooling water inlets of at least 2 (preferably all) of the first heat removal tubes are merged into a cooling water inlet header in the heat removal section, and/or, where there are a plurality of the first heat removal tubes, the cooling water outlets of at least 2 (preferably all) of the first heat removal tubes are merged into a cooling water outlet header in the heat removal section, and/or, where there are a plurality of second heat removal tubes, the cooling water inlets of at least 2 (preferably all) of the second heat removal tubes are merged into a cooling water inlet header in the heat removal section, and/or, where there are a plurality of second heat removal tubes, the cooling water outlets of at least 2 (preferably all) of the second heat removal tubes are merged into a cooling water outlet header in the heat removal section.

8. A fluidized bed reactor, **characterized in that** it comprises a head, a dilute phase zone, a heat removal section, a pre-reaction section and a cone from top to bottom in sequence, wherein a heat removal tube set according to claim 1 is arranged in the heat removal section.

9. A method for controlling the temperature of the fluidized bed reactor according to claim 8, comprising the step of switching the first heat removal tube (where there are a plurality of first heat removal tubes, all of the first heat removal tubes) to the second heat removal tube (where there are a plurality of second heat removal tubes, all of the second heat removal tubes) during the reaction process, so as to maintain the reaction temperature of the fluidized bed reactor substantially constant (preferably, the absolute value of the amplitude of variation is 0.1-1 °C, preferably 0.1-0.5 °C).

10. A method for producing an unsaturated nitrile, comprising the step of subjecting an olefin (such as propylene) to an ammoxidation reaction in a fluidized bed reactor according to claim 8 to obtain an unsaturated nitrile (such as acrylonitrile).

11. A method for producing an unsaturated nitrile, comprising the step of subjecting an olefin (such as propylene) to an ammoxidation reaction in a fluidized-bed reactor to obtain an unsaturated nitrile (such as acrylonitrile), wherein the temperature of the fluidized-bed reactor is controlled by the method for controlling the temperature according to claim 9.

12. The method according to claim 10 or 11, wherein the molar ratio of propylene/ammonia/air (calculated as molecular oxygen) is 1: 1.1-1.3: 1.8-2.0, the reaction temperature is 420-440 °C, the reaction pressure (gauge pressure) is 0.03-0.14 MPa, and the weight hourly space velocity of the catalyst is 0.04-0.15 $h^{-1}$.

Fig. 1

Fig. 2

Fig. 3

Fig. 4

Fig. 5

## INTERNATIONAL SEARCH REPORT

| International application No. |
|---|
| **PCT/CN2022/085769** |

**A. CLASSIFICATION OF SUBJECT MATTER**

B01J 8/24(2006.01)i; C07C 253/26(2006.01)i; C07C 255/08(2006.01)i; F28D 13/00(2006.01)i

According to International Patent Classification (IPC) or to both national classification and IPC

**B. FIELDS SEARCHED**

Minimum documentation searched (classification system followed by classification symbols)

B01J, C07C, F28D

Documentation searched other than minimum documentation to the extent that such documents are included in the fields searched

Electronic data base consulted during the international search (name of data base and, where practicable, search terms used)

CNTXT, ENTXT, ENTXTC, WPABS, WPABSC, VEN, DWPI, CJFD, CNKI: 流化床, 撤热, 除热, 冷却, 丙烯, 氨氧化, fluid +, bed, cool+, remov+, heat, cool+, propylene, ammoxidat+

**C. DOCUMENTS CONSIDERED TO BE RELEVANT**

| Category* | Citation of document, with indication, where appropriate, of the relevant passages | Relevant to claim No. |
|---|---|---|
| A | CN 106492711 A (CHINA PETROLEUM & CHEMICAL CORPORATION et al.) 15 March 2017 (2017-03-15) <br> description, paragraphs [0002]-[0009], and figures 1-5B | 1-12 |
| A | CN 101507907 A (ASAHI CHEMICAL CORP.) 19 August 2009 (2009-08-19) <br> entire document | 1-12 |
| A | CN 104941529 A (INEOS EUROPE AG) 30 September 2015 (2015-09-30) <br> entire document | 1-12 |
| A | CN 102010350 A (SINOPEC NINGBO ENGINEERING CO., LTD. et al.) 13 April 2011 (2011-04-13) <br> entire document | 1-12 |
| A | US 6143915 A (UOP L.L.C.) 07 November 2000 (2000-11-07) <br> entire document | 1-12 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **29 June 2022** | **06 July 2022** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2022/085769**

| Patent document cited in search report | | | Publication date (day/month/year) | Patent family member(s) | | | Publication date (day/month/year) |
|---|---|---|---|---|---|---|---|
| CN | 106492711 | A | 15 March 2017 | None | | | |
| CN | 101507907 | A | 19 August 2009 | CN | 101507907 | B | 25 January 2012 |
| CN | 104941529 | A | 30 September 2015 | TW | 201544181 | A | 01 December 2015 |
| | | | | EA | 201691960 | A1 | 28 February 2017 |
| | | | | JP | 2017512640 | A | 25 May 2017 |
| | | | | KR | 20160140835 | A | 07 December 2016 |
| | | | | WO | 2015153452 | A2 | 08 October 2015 |
| | | | | EP | 3126773 | A2 | 08 February 2017 |
| | | | | TR | 201911130 | T4 | 21 August 2019 |
| CN | 102010350 | A | 13 April 2011 | CN | 102010350 | B | 18 December 2013 |
| US | 6143915 | A | 07 November 2000 | US | 6649130 | B1 | 18 November 2003 |

Form PCT/ISA/210 (patent family annex) (January 2015)